# EUROPÄISCHE PATENTSCHRIFT

(11) **EP 1 590 027 B1**
(45) Veröffentlichungstag und Bekanntmachung des Hinweises auf die Patenterteilung: **09.09.2009**
(21) Anmeldenummer: 03795767.7
(22) Anmeldetag: 19.12.2003
(51) Int. Cl.: A61M 15/00

(54) **Vorrichtung zum einnehmen von pulverförmigen, körnigen oder granulatförmigen Substanzen**
Device for swallowing powder granular or granulated substances
Dispositif pour ingérer des substances pulvérulentes, granuleuses ou granulées

(30) Priorität: 03.01.2003 DE 10300032
(43) Veröffentlichungstag der Anmeldung: 02.11.2005
(73) Patentinhaber: Braunform GmbH, 79353 Bahlingen (DE)
(72) Erfinder: BELLER, Klaus-Dieter, 79341 Kenzingen (DE)
(74) Vertreter: Goy, Wolfgang
(86) Internationale Anmeldenummer: PCT/DE2003/004194
(87) Internationale Veröffentlichungsnummer: WO 2004/060458

(56) Entgegenhaltungen:
- DE-A- 19 855 851
- FR-A- 2 667 790
- FR-A- 2 701 653

## Beschreibung

Die Erfindung betrifft eine Vorrichtung zum Einnehmen von pulverförmigen, körnigen oder granulatförmigen Substanzen nach dem Oberbegriff des Anspruchs 1.

Eine Anwendungsform der erfindungsgemäßen Vorrichtung zum Einnehmen speziell von pulverförmigen Substanzen ist ein sogenannter Pulverinhalator. Eine weitere Anwendungsform sind Vorrichtungen zum Einnehmen speziell von körnigen oder granulatförmigen Substanzen wie beispielsweise Nahrungsergänzungsmittel. Diese zeichnen sich dadurch aus, daß sie relativ grobkörnig sind und daher nicht inhaliert werden. Diese Substanzen werden beispielsweise oral zugeführt, indem sie dem Mund zugeführt werden. Gleichermaßen ist es aber auch denkbar, daß diese körnigen oder granulatförmigen Substanzen Getränken oder Nahrungsmitteln aus dem erfindungsgemäßen Dosierspender zugeführt werden können.

Der erfindungsgemäße treibgasfreie Pulverinhalator dient zur inhalativen Applikation von pulverförmigen, feststofflichen Arzneimitteln oder Nahrungsergänzungsmitteln. Die Pulverinhalatoren setzten das Aerosol durch den Inhalationsvorgang frei, wobei die Energie für die Dispergierung durch den inspiratorischen Fluß gewonnen wird. Die pulverförmige Substanz ist dabei in einem Vorratsbehältnis, nämlich in einem Vorratsbehälter, in einer Kapsel oder in einem Blister enthalten. Bei den Inhalationspulvern handelt es sich dabei um galenische Produkte, welche auf den jeweiligen Inhalator speziell entwickelt werden. Je nach Art des Pulverinhalators wird der reine Wirkstoff eingesetzt oder der Wirkstoff mit einem Träger (unbedenklicher Hilfsstoff, meistens Laktose oder Glukose) für adhärierte Wirkstoffpartikel. Eine weitere Möglichkeit, zuverlässige Pulverinhalatoren zu bereitzustellen, besteht darin, den mikronisierten Wirkstoff zu weichen sowie leicht zerstörbare Agglomerate aufzubauen.

Der Patient atmet bei den derzeit bekannten Pulverinhalatoren ein feines Pulver ein, wobei der Patient die Energie für die Erzeugung des Aerosols mit seinem Atemstrom selbst aufbringt. Dies hat den Vorteil, daß keine Koordinationsprobleme zwischen Freisetzung und Einatmung entstehen. Der Wirkstoff wird in einem beispielsweise spiralig geführten Luftstrom verwirbelt und gelangt mit dem Luftstrom direkt in die Bronchien. Allerdings bringt bei akuter Atemnot der Patient bei den derzeit bekannten Pulverinhalatoren aufgrund der Luftkanalwiderstände die für die Pulveraerosolbehandlung erforderliche Einatmungsstärke nicht immer auf.

Die bekannten Pulverinhalatoren sind dadurch gekennzeichnet, daß sie zu jedem Zeitpunkt einen offenen Luftkanal aufweisen, in den Feuchtigkeit und Umgebungsschmutz eindringen kann. Aus diesem Grunde sind die bekannten Pulverinhalatoren mit Schutzkappen sowie Aufbewahrungseinrichtungen als Schutz vor Feuchtigkeit und Umgebungsschmutz ausgestattet. Bei Verlust oder unsachgemäßer Anwendung dieser Schutzeinrichtungen verliert der Pulverinhalator seine volle Funktionsfähigkeit. Nachteilig bei den bekannten Pulverinhalatoren ist, daß der Patient nicht immer vermeidet, auch in der entgegengesetzten, nämlich in der Expirationsrichtung durch den Inhalator auszuatmen. Dadurch neigt der Inhalator infolge der Atemluftfeuchtigkeit zur Verstopfung, da das Pulver verklumpt und im Inhalator im Luftkanal kleben bleibt. Dies führt auch zu erheblichen Dosierungsproblemen. Der Patient muß die Energie für die Erzeugung des Aerosols mit seinem Atemstrom bei der Inspiration selbst aufbringen. Diese Energie ist aber gerade bei Patienten limitiert, und bei akuter Atemnot kann der Patient die für die Pulver-Aerosol-Behandlung notwendige Energie für die erforderliche Atmungsstärke bei den gängigen Inhalatoren nicht ausreichend aufbringen. Die meisten bekannten Pulverinhalatoren weisen keinen optimalen Strömungsverlauf auf, da es aufgrund der baulichen Vorgaben zu Strömungsumlenkungen und Verwirbelungen kommt. Dies bedeutet ein erhöhter Strömungswiderstand und erfordert einen erhöhten Energieaufwand für den Patienten. Schließlich bleiben Anteile des Pulvers an Hinterfangungen haften und in Ruhezonen der Strömung liegen, was zu Lasten der Dosiergenauigkeit geht.

Die körnigen oder granulatförmigen Substanzen werden bisher dadurch verabreicht, daß sie mit einem Löffel aus dem Vorratsbehältnis, herausgenommen werden, um anschließend den Löffel mit der Substanz in den Mund einzuführen oder aber die Substanz in ein Getränk oder in ein Nahrungsmittel zu geben. Dies ist oftmals unhygienisch, da die Substanz ungeschützt frei zugänglich im Vorratsbehältnis ist. Außerdem ist die notwendige Dosiergenauigkeit nicht immer gewährleistet.

Die FR-A-2 701 653 zeigt einen Pulverinhalator der eingangs angegebenen Art. Dabei ist in einem trichterförmigen Vorratsbehälter das Pulver enthalten. Der trichterförmige Vorratsbehälter weist unterseitig eine Öffnung auf. Diese Öffnung wird durch eine Verschiebeeinrichtung verschlossen. In der Öffnungsstellung dieser Verschiebeeinrichtung kommt eine Öffnung unterhalb der Trichteröffnung zu liegen, so daß das Pulver in einen darunter befindlichen Raum rieseln kann. Aufgrund der Kraft einer Feder wird die Verschiebeeinrichtung wieder in die Ausgangsposition zurückbewegt, in welcher die Verschiebeeinrichtung die Öffnung des Vorratsbehälters verschließt. In der hochgeklappten Stellung des Inhalationsrohres ist dieser Raum durch einen am rückseitigen Ende angeformten Zylinderkörper dieses Inhalationsrohres abgesperrt. Die Inhalation des im Raum befindlichen Pulvers erfolgt dadurch, daß das Inhalationsrohr nach unten geklappt wird, so daß der Durchgangskanal des Inhalationsrohres in Verbindung mit dem Raum gelangt. In dieser Gebrauchsstellung kann die Benutzungsperson das Pulver durch die Durchgangsöffnung des heruntergeklappten Inhalationsrohres einsaugen. - Der Nachteil bei diesem Pulverinhalator besteht darin, daß zunächst die Verschiebeeinrichtung betätigt werden muß, damit Pulver aus dem Vorratsbehälter in den Bereich des Inhalationsrohres, nämlich in den Raum gelangt. Anschließend muß dann das Inhalationsrohr nach unten verschwenkt werden, damit das im Raum befindliche Pulver inhaliert werden kann. Dies stellt insgesamt eine komplizierte Handhabung dar, weil es zweier Betätigungsschritte bedarf.

Die FR-A-2 667 790 zeigt ein Inhalationsgerät, bei welchem ein Inhalationstrichter für die Gebrauchsstellung nach unten verschwenkt werden kann, so daß eine Öffnung im Boden des Inhalationstrichters in räumlicher Verbindung mit der zu inhalierenden Substanz gelangt. Für die Dosierung der Substanz ist ein technisch komplizierter Mechanismus vorgesehen.

Der Erfindung liegt die **Aufgabe** zugrunde, eine Vorrichtung zum Einnehmen von pulverförmigen, körnigen oder granulatförmigen Substanzen der eingangs angegebenen Art zu schaffen, welche einfacher in der Handhabung ist.

Die technische **Lösung** ist gekennzeichnet durch die Merkmale im Kennzeichen des Anspruchs 1.

Die Grundidee der erfindungsgemäßen Vorrichtung zum Einnehmen von pulverförmigen, körnigen oder granulatförmigen Substanzen, insbesondere des erfindungsgemäßen Pulverinhalators besteht darin, daß nur im inhalationsbereiten Zustand des Gerätes zur Führung des Luftstromes ein durchgängig ausgebildeter Luftkanal mit venturirohrartigem oder glattem Profil zur Verfügung steht, während im nicht inhalationsbereiten Zustand der Luftkanal gänzlich blockiert ist, so daß keinerlei Luft im Eingangsbereich und keinerlei pulvertragende Luft im Ausgangsbereich, nämlich im Zuführrohr, angesaugt werden kann. Die Grundidee der technischen Realisierung besteht darin, daß das Zuführrohr am mundfernen Ende drehbar gelagert ist. Dabei sind in der Nichtgebrauchsstellung der Vorrichtung sämtliche Öffnungen verschlossen, während sie in der Gebrauchsstellung geöffnet sind. Vor allem kann bei einem Pulverinhalator der Luftstrom geradlinig ausgebildet sein. Damit ist die Gefahr von Turbulenzen sowie Strömungswiderständen auf ein Minimum reduziert. Denn bei der Herstellung und Applikation von Pulveraerosolen spielt die Adhäsion und die Reibung eine bedeutende Rolle. Während der Applikation des Pulveraerosols müssen Adhäsion und Reibung zwischen Arzneimittel und Hilfsmittel überwunden werden. Aber gleichzeitig treten diese Kräfte zwischen den Pulverteilchen und den Pulverinhalatorflächen auf. Deswegen ist ein geradliniges Profil des Luftstromes von Vorteil. Somit ermöglicht ausschließlich nur zum Zeitpunkt der Anwendung ein entsprechend geformter Luftkanal einen optimalen Strömungsverlauf, und bei zwangsläufiger Synchronisation zwischen Deponieren des Pulvers und Inhalation wird das portionierte pulverförmige Arzneimittel mitgenommen und auf der nachfolgenden Diffusorstrecke hinreichend zerstäubt. Im geschlossenen Zustand hingegen ist die Eingangsöffnung sowie Austrittsöffnung gegenüber Feuchtigkeit und Staub automatisch verschlossen. Selbst bei akuter Atemnot kann der Patient noch die für die Pulver-Aerosolbehandlung mit dem Inhalator erforderliche Atmungsstärke aufbringen, da geradlinige Luftströmungskanäle realisierbar sind. Die synchron arbeitende Dosier- und Freigabeeinrichtung ermöglicht die Minimierung der Bauteile und schließt das gesamte innere System des Gerätes bei Nichtgebrauch luft- und wasserdicht ab. Der Patient öffnet den Luftströmungskanal in dem Gerät mit einer einzigen Bewegung des Zuführrohres. Hierbei wird das Pulver deponiert und kann sofort inhaliert werden. Nach der Inhalation schließt der Patient das System wiederum mit einer einzigen Bewegung durch Verschwenken des Zuführrohres. Hierbei wird aus dem Vorratsbehältnis die nächste Dosis portioniert und das System luftdicht verschlossen. Durch das Verschwenksystem wird insgesamt eine zwangsweise richtige Handhabung für die Einnahmesicherheit gewährleistet. Ein Einwegeventil kann auf der Einstromseite für absolute Dichtigkeit sorgen. Da für die Inbetriebnahme des Pulverinhalators außer dem Zuführrohr keine weiteren Einrichtungen zu bewegen sind, bleibt aufgrund der Geradlinigkeit des Luftströmungskanals beim Inhalationsvorgang der größte Teil der Inspirationsenergie auch tatsächlich für die Inspiration erhalten. Da nur wenige mechanische Teile an dem Inhalator vorhanden sind und der Luftstrom nahezu geradlinig geführt wird, bleiben nur geringe Anteile des Pulvers in der Vorrichtung, d.h. in dem Luftkanal haften. Dies erhöht die Dosiergenauigkeit. Mit der Mitnahme durch den Luftstrom wird das Pulver gleichmäßig verteilt und deagglomeriert. Der Luftkanal hat bevorzugt die Form eines Venturirohres bzw. einer Lavaldüse. Zusätzlich werden beim Transport des Pulvers und Mitnahme durch den Luftstrom gegebenenfalls größere Pulverpartikel durch schraubenförmige Lamellen zerschlagen und damit zerkleinert. In den Luftkanal können beliebig geformte mechanische Widerstände / Desagglomeratoren positioniert werden, um so den Luftkanal bedarfsgerecht sowie spezifikationsgerecht zu variieren. Der Luftkanal kann somit je nach Anforderung des Pulvers im Atemzugswiderstand frei variiert werden. Die Ausführungen des Luftkanals können im Querschnitt rund oder oval sein und über die gesamte Länge den gleichen Querschnitt aufweisen. Der Einlaß und Auslaß kann beispielsweise trichterförmig sein. Durch das Venturirohr ist eine optimale Strömung und Beschleunigung des inspirierten Luftstromes einschließlich Mitnahme und Zerstäubung des pulverförmigen Arzneimittels gewährleistet. Insbesondere ist dadurch eine zwangsweise komplette Entleerung gewährleistet. Die freie Gestaltung des Luftkanals ermöglicht die Anpassung des Inhalators an die verschiedensten inhalationsfähigen Arzneimittel. So können trockene pulverförmige Arzneimittel im Luftstrom in partikeldefinierter Größe dispergiert werden. Der erfindungsgemäße Inhalator ist insgesamt für einen längeren Gebrauch geeignet, da durch die Bauweise der gesamte Luftkanal mittels eines Pfeifenreinigers leicht zu reinigen ist und hygienische Probleme vermieden werden. Der Inhalator besteht bei Vollfunktion und einfachster Form aus wenigen Spritzgußteilen ohne die Verwendung von Federn und Hebeln. Vorstehend wurde die Erfindung anhand eines Pulverinhalators beschrieben. Die entsprechenden Vorteile ergeben sich auch bei körnigen oder granulatförmigen Substanzen, welche jedoch nicht inhaliert werden, sondern vielmehr aus der erfindungsgemäßen Vorrichtung dosiert herausrieseln und entsprechend verwendet werden können. Der Unterschied zum Pulverinhalator besteht darin, daß kein durchgängiger Luftstrom zum Inhalieren durch den Benutzer erzeugt wird.

Eine erste Anwendung der erfindungsgemäßen Vorrichtung schlägt Anspruch 2 vor. Es wird dabei von einem Reservoir für die Substanz ausgegangen, aus dem mehrere Dosiereinheiten portioniert werden können.

Die Grundidee des Pulverinhaltors in Anspruch 3 besteht darin, daß der Zylindermantel des Zuführrohres eine Durchgangsöffnung in Form einer Bohrung aufweist, deren Höhe und Durchmesser das Dosiervolumen bestimmt und einer gegenüberliegenden Bohrung im zentralen Zylinderkörper entspricht. Im geschlossenen Zustand liegt die Durchgangsöffnung des Zylindermantels genau unterhalb der Auslaßöffnung des Vorratsbehältnisses, so daß das Arzneimittel dosiert wird. Im heruntergeklappten Zustand des Zuführrohres rutscht dann das Arzneimittel durch die Verbindungsbohrung im Zylinderkörper in die Durchgangsbohrung und damit exakt in den Luftkanal, so daß das Arzneimittel bei Inspiration inhaliert werden kann. Das Vorratsbehältnis kann zur Erhöhung der Wirtschaftlichkeit auswechselbar sein.

Die Weiterbildung hiervon gemäß Anspruch 4 schlägt ein Einwegventil im Luftkanal vor, so daß der Patient das Pulver nur einatmen, nicht aber aus dem Inhalator hinausblasen kann.

Die Weiterbildung gemäß Anspruch 5 betrifft keinen Pulverinhalator, sondern eine Vorrichtung zum Dosieren von körnigen oder granulatförmigen Substanzen. Die Grundidee hier besteht darin, daß die Substanz durch die Auslaßöffnung des Voratsbehältnisses hindurch in die Durchgangsöffnung im Zylinder rieselt, welche eine Dosierkammer definiert. Nach dem Verschwenken des Zuführrohres in die Gebrauchsstellung rieselt die Substanz aus diesem Dosierraum in die Durchgangsbohrung des Zylinderkörpers und von dort in das Zuführrohr.

Eine zweite Anwendung der erfindungsgemäßen Vorrichtung schlägt Anspruch 6 vor. Es handelt sich hier um einen Einzeldosis-Applikator unter Verwendung einer Kapsel. Bei diesem Einmaldosierer liegt die Kapsel in dem Zylinderkörper der Drehachse für das Zuführrohr. Durch das Herunterklappen des Zuführrohres werden die Kuppen der Kapsel mittels eines Schneidmessers abgeschnitten. Dadurch ist eine komplette Entleerung der Kapsel möglich. Es gibt darüber hinaus keine Splitter. Im Gegensatz zu anderen Geräten ist deshalb kein Schutzsieb notwendig, welches den Atmungswiderstand erhöht. Eine ungeöffnete Kapsel kann nur im Nichtgebrauchszustand des Zuführrohres eingelegt werden. Dies gilt gleichermaßen auch für die Entsorgung einer entleerten Kapsel. Dadurch ist eine zwangsweise Synchronisation realisiert, indem das Aufschneiden der Kapsel und das Aktivieren in der entsprechenden Verschwenkstellung des Zuführrohres erfolgt, während in der Nichtgebrauchsstellung des Zuführrohres ein luft- und wasserdichter Abschluß realisiert ist.

Drei Ausführungsbeispiele einer erfindungsgemäßen Vorrichtung zum Einnehmen von pulverförmigen, körnigen oder granulatförmigen Substanzen werden nachfolgend anhand der Zeichnungen beschrieben. In diesen zeigt:

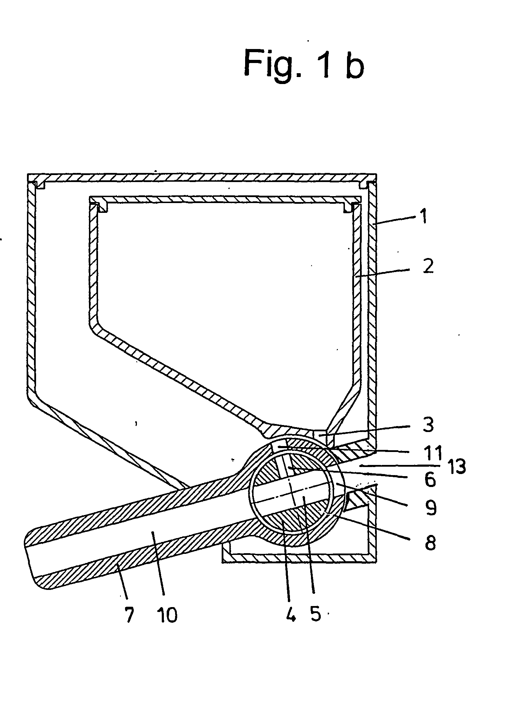

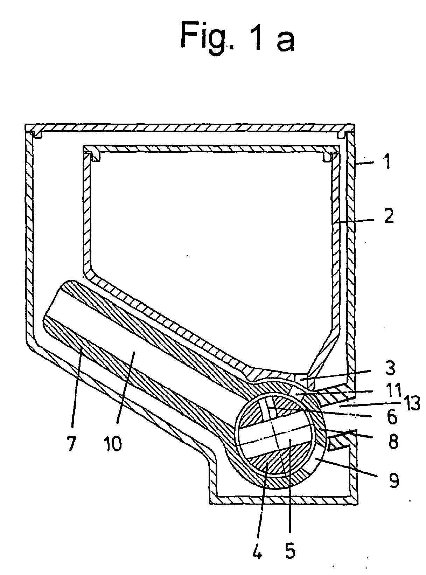

- Fig. 1 a: eine erste Ausführungsform eines Pulverinhalators für mehrere Dosiereinheiten (sogenannter Multidose) in der Nichtgebrauchsstellung;
- Fig. 1 b: den Pulverinhalator in Fig. 1a in der Gebrauchsstellung;
- Fig. 2a: eine zweite Ausführungsform eines Pulverinhalators für eine Einmaldosierung (sogenannter Singledose) in der Nichtgebrauchsstellung;
- Fig. 2b: den Pulverinhalator in Fig. 2a in der Gebrauchsstellung;
- Fig. 3a: eine dritte Ausführungsform als Vorrichtung zum Einnehmen von körnigen oder granulatförmigen Substanzen für mehrere Dosiereinheiten (sogenannter Multidose) in der Nichtgebrauchsstellung;
- Fig. 3b: die Vorrichtung in Fig. 3a in der Gebrauchsstellung.

Der Pulverinhalator der ersten Ausführungsform in Fig. 1a und 1b weist ein Gehäuse 1 auf, in welchem sich ein im wesentlichen trichterförmiges Vorratsbehältnis 2 für ein Pulver befindet. Dieses Vorratsbehältnis 2 weist an der untersten Stelle eine Auslaßöffnung 3 auf.

In dem Gehäuse ist weiterhin ein Zylinderkörper 4 fest angeordnet. Dieser weist eine radiale Durchgangsbohrung 5 auf. Von dieser Durchgangsbohrung 5 geht quer eine ebenfalls durchgehende Verbindungsbohrung 6 nach oben hin ab.

Auf dem Zylinderkörper 4 ist ein Zuführrohr 7 in Form eines Inhalationsrohres drehbar gelagert. Zu diesem Zweck weist das hintere Ende dieses Zuführrohres 7 einen Zylindermantel 8 auf, mittels welchem die Verschwenklagerung auf dem Zylinderkörper 4 realisiert ist. Dieser Zylindermantel 8 weist eine Öffnung 9 auf, welche auf der Achse der Innenöffnung 10 des Zuführrohres 7 liegt. Außerdem weist der Zylindermantel 8 eine Durchgangsöffnung 11 auf.

Schließlich besitzt das Gehäuse 1 einen nach innen ragenden Lufteinlaß 13.

### Die Funktionsweise ist wie folgt:

In der Nichtgebrauchsstellung (Fig. 1a) befindet sich die Durchgangsöffnung 11 des Zylindermantels 8 unterhalb der Auslaßöffnung 3 des Vorratsbehälters 2. Das Pulver rieselt in die Durchgangsöffnung 11, welche damit eine Dosiereinheit definiert. In dieser Stellung ist die Durchgangsbohrung 5 sowie die Verbindungsbohrung 6 des Zylinderkörpers 4 durch den Zylindermantel 8 abgedichtet geschlossen.

Zum Überführen des Pulverinhalators in die Gebrauchsstellung (Fig. 1 b) wird das Zuführrohr 7 nach unten verschwenkt. Dadurch gelangt die Durchgangsöffnung 11 (mit der Dosiereinheit an Pulver) oberhalb der Verbindungsbohrung 6 im Zylinderkörper 4, so daß das Pulver durch die Verbindungsbohrung 6 hindurch in die Durchgangsbohrung 5 rieselt. Entsprechend definieren die Innenöffnung 10 des Zuführrohres 7, die Durchgangsbohrung 5 des Zylinderkörpers 4, die Öffnung 9 des Zylindermantels 8 des Zuführrohres 7 sowie der Lufteinlaß 13 des Gehäuses 1 einen miteinander verbundenen, durchgägngigen sowie abgedichteten Durchgangskanal. Durch eine Inhalation durch den Benutzer wird das Pulver mitgerissen und vom Patienten eingeatmet. Das Inhaltaionsrohr 7 kann grundsätzlich als Mundrohr oder als Nasenrohr konzipiert sein.

Nach der Inhalation wird das Zuführrohr 7 wieder nach oben verschwenkt (Fig. 1a), so daß eine neue Dosis an Pulver aus dem Vorratsbehälter 2 durch die Auslaßöffnung 3 hindurch in die Durchgangsöffnung 11 im Zylindermantel 8 gelangt.

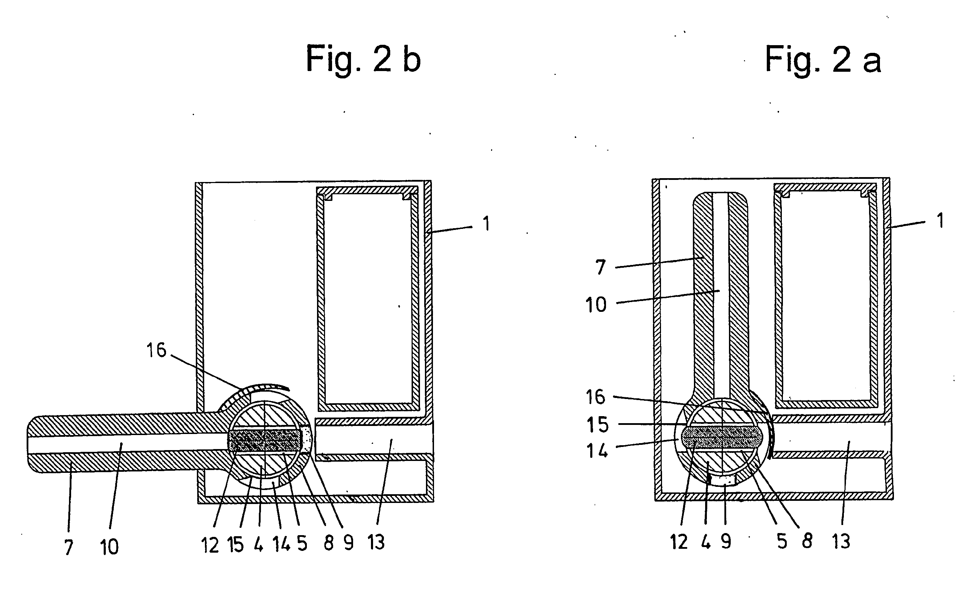

Die zweite Ausführungsform in Fig. 2a und 2b geht von einem Vorratsbehältnis in Form einer Kapsel 12 für eine Einmaldosierung aus. Vom Grundprinzip her ist diese Ausführungsvariante des Pulverinhalators gleich dem Grundprinzip des Pulverinhalators in Fig. 1a und 1 b.

So ist ein Zylinderkörper 4 mit einer Durchgangsbohrung 5 vorgesehen (jedoch ohne Verbindungsbohrung 6). Auf diesem Zylinderkörper 4 ist ein Zylindermantel 8 eines Zuführrohres 7 verschwenkbar gelagert. Dieser Zylindermantel 8 weist auf der Achse der Innenöffnung 10 des Zuführrohres 7 eine Öffnung 9 auf. Außerdem weist der Zylindermantel 8 zwei Durchbrechungen 14 mit jeweils einer Schneidkante 15 auf. Die in Fig. 2b obere Durchbrechung 14 weist zusätzlich eine Abdichtnase 16 auf.

### Die Funktionsweise ist wie folgt:

In der Nichtgebrauchsstellung des Pulverinhalators (Fig. 2a) wird eine Kapsel 12 in die Durchgangsbohrung 5 des Zylinderkörpers 4 eingeschoben. Die Innenöffnung 10 des Zuführrohres 7 sowie die Öffnung 9 des Zylindermantels 8 sind durch den Zylinderkörper 4 abgedichtet geschlossen. Außerdem ist der Lufteinlaß 13 des Gehäuses 1 durch die Abdichtnase 16 dicht verschlossen.

Zum Überführen des Pulverinhalators in die Gebrauchsstellung wird das Zuführrohr 7 nach unten geklappt (Fig. 2b). Dabei scheren die beiden Schneidkanten 15 des Zylindermantels 8 die Kuppen der Kapsel 12 ab. Dadurch befindet sich das Pulver im Innern der Kapsel 12 im Luftstrom während des Inhalationsvorganges. Der abgedichtete Luftkanal wird dabei durch die Innenöffnung 10 des Zuführrohres 7, die Durchgangsbohrung 5 im Zylinder 4, die Öffnung 9 im Zylindermantel 8 sowie durch den Lufteinlaß 13 des Gehäuses 1 definiert.

Nach Beendigung der Inhalation wird das Zuführrohr 7 wieder nach oben verschwenkt, so daß die leere Kapsel 12 entnommen werden kann.

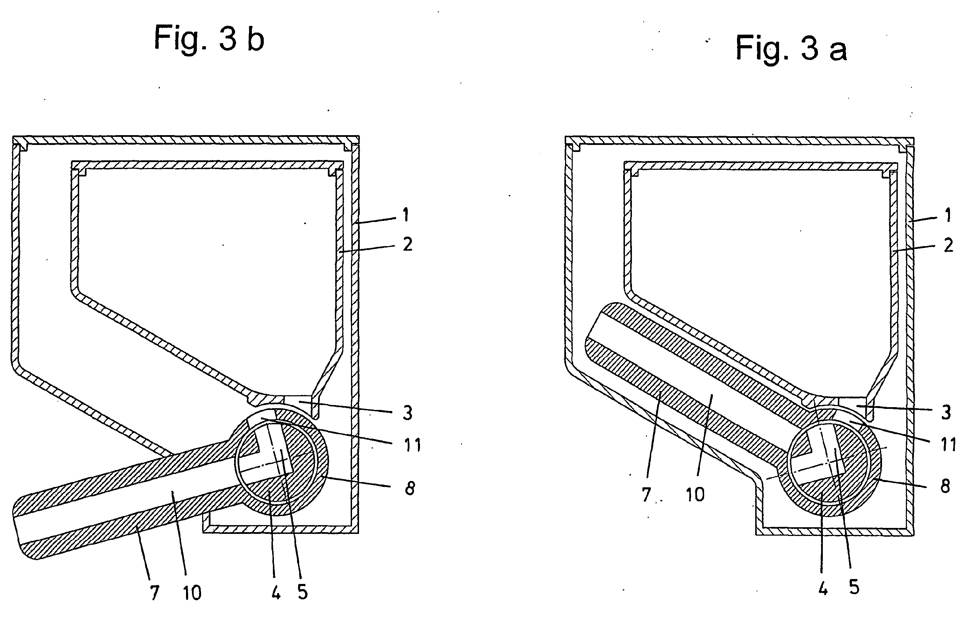

Die dritte Ausführungsform in Fig. 3a und 3b dient zur dosierten Abgabe von körnigen oder granulatförmigen Substanzen, welche sich wiederum im Vorratsbehältnis 2 befinden. Der Unterschied zum Pulverinhalator der Fig. 1a und 1b besteht darin, daß der Zylindermantel 8 nicht die Öffnung 9 aufweist und daß die Durchgangsbohrung 5 im Zylinderkörper 4 winkelförmig ausgebildet ist, wobei diese Durchgangsbohrung 5 vom oberen Einlaß bis zum unteren Auslaß bezüglich der Erdhorizontalen ein durchgängiges Gefälle aufweist.

### Die Funktionsweise ist wie folgt:

In dem Vorratsbehältnis 2 befindet sich - wie gesagt - eine körnige oder granulatförmige, rieselförmige Substanz In der Nichtgebrauchsstellung der Vorrichtung (Fig. 3a) ist das Zuführrohr 7 nach oben geklappt, so daß die Durchgangsöffnung 11 des Zylindermantels 8 unterhalb der Auslaßöffnung 3 zu liegen kommt. Die Durchgangsbohrung 5 im Zylinderkörper 4 ist dabei durch den Zylindermantel 8 vollständig geschlossen. In der Durchgangsöffnung 11 des Zylindermantels 8 sammelt sich die körnige oder granulatförmige Substanz.

Nach dem Nachuntenschwenken des Zuführrohres 7 in die Gebrauchsstellung (Fig. 3b) gelangt die Durchgangsöffnung 6 des Zylindermantels 8 oberhalb des Einlasses der winkeligen Durchgangsbohrung 5. Die in der Durchgangsöffnung 11 des Zylindermantels 8 befindliche Substanz rieselt von oben in die Durchgangsbohrung 5. Aufgrund ihrer Rieselfähigkeit gelangt die Substanz schließlich in die Innenöffnung 10 des Zuführrohres 7, von wo aus die Substanz entweder oral aufgenommen oder einem Getränk oder Nahrungsmittel oder anderweitig direkt zugeführt werden kann.

### Bezugszeichenliste

- 1: Gehäuse
- 2: Vorratsbehältnis
- 3: Auslaßöffnung
- 4: Zylinderkörper
- 5: Durchgangsbohrung
- 6: Verbindungsbohrung
- 7: Zuführrohr
- 8: Zylindermantel
- 9: Öffnung
- 10: Innenöffnung
- 11: Durchgangsöffnung
- 12: Kapsel
- 13: Lufteinlaß
- 14: Durchbrechung
- 15: Schneidkante
- 16: Abdichtnase

## Patentansprüche

1. Vorrichtung zum Einnehmen von pulverförmigen, körnigen oder granulatförmigen Substanzen
mit einem Vorratsbehältnis (2) für die Substanz sowie
mit einem, eine Innenöffnung (10) aufweisenden Zuführrohr (7) zum Zuführen der Substanz,
wobei das Zuführrohr (7) mit seinem hinteren Ende mittels eines angeformten Zylinderteils derart verschwenkbar gelagert ist,
daß in einer Nichtgebrauchsstellung des Zuführrohres (7) die Substanz in dem Vorratsbehältnis (2) luftdicht abgeschlossen ist und
daß beim Verschwenken des Zuführrohres (7) in die Gebrauchsstellung die Substanz in das Zuführrohr (7) gelangt,
**dadurch gekennzeichnet,**
**daß** das Zylinderteil ein Zylindermantel (8) ist, welcher auf einem feststehenden Zylinderkörper (4) verschwenkbar gelagert ist,
**daß** der Zylinderkörper (4) eine Durchgangsbohrung (5) aufweist,
**daß** der Zylindermantel (8) eine Durchgangsöffnung (11) aufweist,
**daß** in der Nichtgebrauchsstellung des Zuführrohres (7) sich die Innenöffnung (10) des Zuführrohres (7) und die Durchgangsöffnung (11) des Zylindermantels (8) nicht im Bereich der Durchgangsbohrung (5) des Zylinderkörpers (4) befinden und
**daß** in der Gebrauchsstellung des Zuführrohres (7) sich die Innenöffnung (10) des Zuführrohres (7) und die Durchgangsöffnung (11) des Zylindermantels (8) im Bereich der Durchgangsbohrung (5) des Zylinderkörpers (4) befinden, in welcher sich die Substanz befindet.

2. Vorrichtung nach Anspruch 1,
**dadurch gekennzeichnet,**
**daß** sich in dem Vorratsbehältnis (2) die Substanz in mehreren Dosiereinheiten befindet, daß das Vorratsbehältnis (2) unterseitig eine Auslaßöffnung (3) aufweist und
**daß** sich die Durchgangsöffnung (11) des Zylindermantels (8) in der Nichtgebrauchsstellung des Zuführrohres (7) unterhalb der Auslaßöffnung (3) des Vorratsbehältnisses (2) und in der Gebrauchsstellung des Zuführrohres (7) in Verbindung mit der Durchgangsbohrung (5) des Zylinderkörpers (4) befindet.

3. Vorrichtung nach Anspruch 2,
**dadurch gekennzeichnet,**
**daß** der Zylinderkörper (4) eine radiale Durchgangsbohrung (5) sowie quer zur Durchgangsbohrung (5) eine nach oben abgehende, durchgehende Verbindungsbohrung (6) aufweist,
wobei die radiale Durchgangsbohrung (5) während des Inhalierens einen durchgängigen Luftstrom definiert, welcher die in der Durchgangsbohrung (5) befindliche Substanz mitreißt,
**daß** der Zylindermantel (8) in der axialen Verlängerung des Zuführrohres (7) eine Öffnung (9) aufweist und
**daß** sich die Durchgangsöffnung (11) des Zylindermantels (8) in der Nichtgebrauchsstellung des Zuführrohres (7) unterhalb der Auslaßöffnung (3) des Vorratsbehältnisses (2) und in der Gebrauchsstellung des Zuführrohres (7) oberhalb der Verbindungsbohrung (6) des Zylinderkörpers (4) befindet.

4. Vorrichtung nach Anspruch 3,
**dadurch gekennzeichnet,**
**daß** ein den Luftstrom definierender Luftkanal ein Einwegventil aufweist.

5. Vorrichtung nach Anspruch 2,
**dadurch gekennzeichnet,**
**daß** der Zylinderkörper (4) eine abgewinkelte Durchgangsbohrung (5) mit einem bezüglich der Erdhorizontalen durchgängigen Gefälle aufweist und
**daß** in dem Zylindermantel (8) die Durchgangsöffnung (11) derart angeordnet und ausgebildet ist,
**daß** sich die Durchgangsöffnung (11) in der Nichtgebrauchsstellung des Zuführrohres (7) unterhalb der Auslaßöffnung (3) des Vorratsbehältnisses (2) bei durch den Zylindermantel (8) geschlossener Durchgangsbohrung (5) befindet und
**daß** sich die Durchgangsöffnung (11) in der Gebrauchsstellung des Zuführrohres (7) oberhalb des Einlasses der Durchgangsbohrung (5) des Zylinderkörpers (4) befindet und wobei die Innenöffnung (10) des Zuführrohres (7) mit dem Auslaß der Durchgangsbohrung (5) in Verbindung steht.

6. Vorrichtung nach Anspruch 1,
**dadurch gekennzeichnet,**
**daß** das Vorratsbehältnis (2) eine Kapsel (12) für eine einzige Dosiereinheit ist,
**daß** die Kapsel (12) in die Durchgangsbohrung (5) des Zylinderkörpers (4) einführbar ist und
**daß** die beiden Enden der Kapsel (12) jeweils derart über die Mantelfläche des Zylinderkörpers (4) überstehen,
**daß** beim Verschwenken des Zuführrohres (7) von der Nichtgebrauchsstellung in die Gebrauchssstellung diese Enden abgeschert werden.

## Claims

1. Device for inhaling or ingesting powdery, granular or granulate substances, with a storage container (2) for the substance and
with a feed pipe (7) having an internal opening (10) for supplying the substance,
wherein the feed pipe (7) is mounted swivellably with its rear end by means of a cylindrical part mounted such that in a non-usage position of the feed pipe (7), the substance is sealed air-tight in the storage container (2), and that on swivelling of the feed pipe (7) into the usage position, the substance enters the feed pipe (7),
**characterised in that**
the cylinder part is a cylinder casing (8) which is swivellably mounted on a fixed cylinder body (4),
**in that** the cylinder body (4) has a passage bore (5),
**in that** the cylinder casing (8) has a passage opening (11),
**in that** in the non-usage position of the feed pipe (7), the internal opening (10) of the feed pipe (7) and the passage opening (11) of the cylinder casing (8) are not situated in the region of the passage bore (5) of the cylinder body (4), and
**in that** in the usage position of the feed pipe (7), the internal opening (10) of the feed pipe (7) and the passage opening (11) of the cylinder casing (8) are situated in the region of the passage bore (5) of the cylinder body (4) in which the substance is present.

2. Device according to claim 1, **characterised in that** the substance is present in the storage container (2) in multiple dosage units,
**in that** the storage container (2) has an outlet opening (3) on the underside, and
**in that** the passage opening (11) of the cylinder casing (8) in the non-usage position of the feed pipe (7) is situated below the outlet opening (3) of the storage container (2), and in the usage position of the feed pipe (7) is connected with the passage bore (5) of the cylinder body (4).

3. Device according to claim 2, **characterised in that** the cylinder body (4) has a radial passage bore (5) and transverse to the passage bore (5) a through connecting bore (6) leading upwards, wherein the radial passage bore (5) during inhalation defines a through air flow which carries with it the substance which is present in the passage bore (5),
**in that** the cylinder casing (8) has an opening (9) in the axial extension of the feed pipe (7), and
**in that** the passage opening (11) of the cylinder casing (8) in the non-usage position of the feed pipe (7) is situated below the outlet opening (3) of the storage container (2), and in the usage position of the feed pipe (7) is situated above the connecting bore (6) of the cylinder body (4).

4. Device according to claim 3, **characterised in that** an air channel defining the air flow has a one-way valve.

5. Device according to claim 2, **characterised in that** the cylinder body (4) has an angled passage bore (5) with a through fall in relation to the horizontal, and
**in that** in the cylinder casing (8) the passage opening (11) is arranged and formed such that the passage opening (11) in the non-usage position of the feed pipe (7) is situated below the outlet opening (3) of the storage container (2) with the through bore (5) closed by the cylinder casing (8), and
**in that** the passage opening (11) in the usage position of the feed pipe (7) is situated above the inlet of the passage bore (5) of the cylinder body (4), and,
wherein the internal opening (10) of the feed pipe (7) is connected with the outlet of the passage bore (5).

6. Device according to claim 1, **characterised in that** the storage container (2) is a capsule (12) for a single dose unit,
**in that** the capsule (12) can be introduced into the passage bore (5) of the cylinder body (4), and
**in that** the two ends of the capsule (12) each protrude over the casing surface of the cylinder body (4),
**in that** on swivelling of the feed tube (7) from the non-usage position to the usage position, these ends are sheared off.

## Revendications

1. Dispositif pour la prise de substances pulvérulentes, granuleuses ou granulées, comprenant
un récipient de réserve (2) pour la substance,
ainsi qu'un tube d'amenée (7) pourvu d'une ouverture intérieure (10), afin de délivrer ladite substance,
ledit tube d'amenée (7) étant monté à pivotement par son extrémité postérieure, au moyen d'une partie cylindrique solidaire,
de telle sorte que la substance soit confinée hermétiquement dans le récipient de réserve (2), dans une position de non-utilisation dudit tube d'amenée (7),
et que ladite substance parvienne dans ledit tube d'amenée (7) lors du pivotement dudit tube d'amenée (7) jusqu'à la position d'utilisation,
**caractérisé par le fait**
**que** la partie cylindrique est une enveloppe cylindrique (8) montée pivotante sur un corps cylindrique fixe (4) ;
**que** ledit corps cylindrique (4) présente un perçage traversant (5) ;
**que** ladite enveloppe cylindrique (8) comporte une ouverture de passage (11) ;
**que**, dans la position de non-utilisation du tube d'amenée (7), l'ouverture intérieure (10) dudit tube d'amenée (7) et l'ouverture de passage (11) de l'enveloppe cylindrique (8) ne sont pas disposées dans la région du perçage traversant (5) du corps cylindrique (4) ; et
**que**, dans la position d'utilisation du tube d'amenée (7), l'ouverture intérieure (10) dudit tube d'amenée (7) et l'ouverture de passage (11) de l'enveloppe cylindrique (8) sont disposées dans la région un perçage traversant (5) du corps cylindrique (4), dans lequel la substance se trouve.

2. Dispositif selon la revendication 1,
**caractérisé par le fait**
**que** le récipient de réserve (2) renferme la substance en plusieurs unités de dosage ;
**que** ledit récipient de réserve (2) comporte une ouverture de sortie (3) en partie basse ; et
**que** l'ouverture de passage (11) de l'enveloppe cylindrique (8) se trouve au-dessous de l'ouverture de sortie (3) du récipient de réserve (2) dans la position de non-utilisation du tube d'amenée (7), et est en liaison avec perçage traversant (5) du corps cylindrique (4) dans la position d'utilisation dudit tube d'amenée (7).

3. Dispositif selon la revendication 2,
**caractérisé par le fait**
**que** le corps cylindrique (4) comporte un perçage traversant radial (5) et, transversalement vis-à-vis dudit perçage traversant (5), un conduit ininterrompu de jonction (6) bifurquant vers le haut,
ledit perçage traversant radial (5) définissant, au cours de l'inhalation, un courant d'air continu charriant, avec lui, la substance située dans ledit perçage traversant (5) ;
**que** l'enveloppe cylindrique (8) présente une ouverture (9) dans le prolongement axial du tube d'amenée (7) ; et
**que** l'ouverture de passage (11) de l'enveloppe cylindrique (8) se trouve au-dessous de l'ouverture de sortie (3) du récipient de réserve (2) dans la position de non-utilisation du tube d'amenée (7), et au-dessus du conduit de jonction (6) du corps cylindrique (4) dans la position d'utilisation dudit tube d'amenée (7).

4. Dispositif selon la revendication 3,
**caractérisé par le fait**
**qu'**un canal de circulation d'air, définissant le flux d'air, comporte un clapet unidirectionnel.

5. Dispositif selon la revendication 2,
**caractérisé par le fait**
**que** le corps cylindrique (4) présente un perçage traversant (5) coudé, à pente continue vis-à-vis de l'horizontale ; et
**que** l'ouverture de passage (11) est agencée et réalisée, dans l'enveloppe cylindrique (8), de telle sorte que l'ouverture de passage (11) se trouve au-dessous de l'ouverture de sortie (3) du récipient de réserve (2) dans la position de non-utilisation du tube d'amenée (7), lorsque le perçage traversant (5) est fermé par l'enveloppe cylindrique (8),
et **que** l'ouverture de passage (11) se trouve au-dessus de l'admission du perçage traversant (5) du corps cylindrique (4) dans la position d'utilisation dudit tube d'amenée (7),
l'ouverture intérieure (10) dudit tube d'amenée (7) étant en liaison avec la sortie dudit perçage traversant (5).

6. Dispositif selon la revendication 1,
**caractérisé par le fait**
**que** le récipient de réserve (2) est une capsule (12) destinée à une unique unité de dosage ; que ladite capsule (12) peut être insérée dans le perçage traversant (5) du corps cylindrique (4) ; et
**que** les deux extrémités de ladite capsule (12) font respectivement saillie, au-delà de la surface périphérique dudit corps cylindrique (4),
de telle sorte que ces extrémités soient cisaillées lors du pivotement dudit tube d'amenée (7) lorsqu'on passe de la position de non-utilisation à la position d'utilisation.
